(19) Europäisches Patentamt / European Patent Office / Office européen des brevets

(11) **EP 4 523 712 A1**

(12) **EUROPEAN PATENT APPLICATION**
published in accordance with Art. 153(4) EPC

(43) Date of publication:
**19.03.2025   Bulletin 2025/12**

(21) Application number: **23819802.2**

(22) Date of filing: **05.06.2023**

(51) International Patent Classification (IPC):
**A61L 27/14** (2006.01)      **A61L 27/12** (2006.01)
**A61L 27/46** (2006.01)      **A61L 27/52** (2006.01)

(52) Cooperative Patent Classification (CPC):
**A61L 27/12; A61L 27/14; A61L 27/46; A61L 27/52**

(86) International application number:
**PCT/JP2023/020821**

(87) International publication number:
**WO 2023/238822 (14.12.2023 Gazette 2023/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **10.06.2022   JP 2022094413**

(71) Applicants:
 • National University Corporation
   **Hokkaido University**
   **Hokkaido 060-0808 (JP)**
 • **Niterra Co., Ltd.**
   **Nagoya-shi, Aichi 461-0005 (JP)**
   Designated Contracting States:
   **AL AT BG CH CZ DE DK EE ES FI GB HR HU IS IT
   LI LU MK NO PL PT RO RS SE SK TR**

(72) Inventors:
 • **KUROKAWA, Takayuki**
   **Sapporo-shi, Hokkaido 060-0808 (JP)**
 • **GONG, Jian Ping**
   **Sapporo-shi, Hokkaido 060-0808 (JP)**
 • **NONOYAMA, Takayuki**
   **Sapporo-shi, Hokkaido 060-0808 (JP)**
 • **YASUDA, Kazunori**
   **Sapporo-shi, Hokkaido 060-0808 (JP)**
 • **IWATA, Masaya**
   **Nagoya-shi, Aichi 461-0005 (JP)**
 • **MURAI, Ryota**
   **Nagoya-shi, Aichi 461-0005 (JP)**

(74) Representative: **Patentanwaltskanzlei WILHELM
   & BECK**
   **Prinzenstraße 13**
   **80639 München (DE)**

(54) **ARTIFICIAL CARTILAGE IMPLANT AND MANUFACTURING METHOD THEREFOR**

(57)    The present invention addresses the problem of providing an artificial cartilage implant which is changed little even when used in a living body and can be stably used over a long period of time. The artificial cartilage implant for solving the problem has: a bone implant part which includes a polymer gel and hydroxyapatite particles dispersed at least near the surface of the polymer gel; and an artificial cartilage part which includes a polymer gel, wherein, when the cross section of the polymer gel of the artificial cartilage part is observed, the hydroxyapatite particles are not dispersed in a region spanning from the surface to a depth of 1,000 $\mu$m, or the content of the hydroxyapatite particles in the region spanning from the surface to a depth of 1,000 $\mu$m is less than 0.1 wt% in the case in which the hydroxyapatite particles are dispersed in the region.

EP 4 523 712 A1

FIG. 1A

FIG. 1B

FIG. 1C

FIG. 1D

FIG. 1E

## Description

Technical Field

The present invention relates to an artificial cartilage implant and a method for manufacturing the artificial cartilage implant.

Background Art

Polymer gels have a structure such that a resin is crosslinked in a three-dimensional shape, and their use in vivo has been considered in recent years due to their high elasticity and ease of obtaining good biocompatibility. For example, Patent Literature (hereinafter, referred to as PTL) 1 and PTL 2 describe the application of a composite, in which hydroxyapatite particles are dispersed in a polymer gel, to a living body. Further, PTL 3 describes that calcium ions dispersed inside a polymer gel are reacted with phosphorus in a living body to form hydroxyapatite inside the polymer gel. Further, PTL 4 describes using a composite, in which a hydrogel is covered with hydroxyapatite, as an artificial bone.

Further, Non-Patent Literature (hereinafter, referred to as NPL) 1 describes using a composite in which fine hydroxyapatite particles are dispersed in a polymer gel as a substitute for cartilage.

Citation List

Patent Literature

PTL 1
Japanese Patent Application Laid-Open No. H1-305959
PTL 2
Japanese Patent Application Laid-Open No. 2004-262976
PTL 3
Japanese Patent Application Laid-Open No. 2004-321501
PTL 4
WO1999/058447

Non-Patent Literature

NPL 1
Takayuki Nonoyama, "Double-Network Hydrogels Strongly Bondable to Bones by Spontaneous Osteogenesis Penetration", Advanced Materials, 2016, 28, 6740-6745

Summary of Invention

Technical Problem

In any of the above-described techniques, hydroxyapatite covers the entire surface of the polymer gel, or hydroxyapatite particles are dispersed throughout the entire polymer gel. However, the present inventors have diligently studied the use of such a composite in a portion of a living body where elasticity is required (for example, cartilage), and it has been found that fracture or the like occurs over time. The reason for this is that the inorganic component present in the living body adheres to the hydroxyapatite of the above composite, and the properties of a portion including the hydroxyapatite become close to those of ceramics. As a result, when a load is applied to the portion, it is considered that strain occurs and fracture occurs.

The present invention has been made in view of the above problems. Specifically, an object of the present invention is to provide an artificial cartilage implant that undergoes little change in a living body and can be used stably over a long period of time, and a method for manufacturing the artificial cartilage implant.

Solution to Problem

The present invention provides an artificial cartilage implant that includes: a bone implant section including a polymer gel and hydroxyapatite particles dispersed at least in a vicinity of a surface of the polymer gel; and an artificial cartilage section including a polymer gel, in which

when a cross section of the polymer gel of the artificial cartilage section is observed, no hydroxyapatite particle is distributed in a region from a surface to a depth of 1000 $\mu$m, or in a case where hydroxyapatite particles are distributed in the region, a content of the hydroxyapatite particles at the depth of 1000 $\mu$m from the surface is less than 0.1% by weight.

The present invention provides a method for manufacturing the above artificial cartilage implant, the method including: preparing a polymer gel; and forming the hydroxyapatite particles in a vicinity of a surface of a region corresponding to the bone implant section by sequentially bringing an alkali metal phosphate aqueous solution and a calcium salt aqueous solution into contact with the region corresponding to the bone implant section while protecting a region corresponding to the artificial cartilage section, the region corresponding to the bone implant and the region corresponding to the artificial cartilage section being in the polymer gel.

The present invention also provides a method for manufacturing the above artificial cartilage implant, the method including: preparing a polymer gel; forming the hydroxyapatite particles in a vicinity of a surface of the polymer gel by sequentially bringing an alkali metal phosphate aqueous solution and a calcium salt aqueous solution into contact with the polymer gel; and removing the hydroxyapatite particles in a region corresponding to the artificial cartilage section by bringing an acidic solution into contact with the region corresponding to the artificial cartilage section while protecting a region corresponding to the bone implant section, the region corresponding to the artificial cartilage section and the region corresponding to the bone implant being in the polymer gel after the forming of the hydroxyapatite particles.

Advantageous Effects of Invention

An artificial cartilage implant of the present invention undergoes little change even when the artificial cartilage implant is used in a living body and therefore can be used stably over a long period of time. Further, a manufacturing method of an artificial cartilage implant of the present invention can produce the above artificial cartilage implant by a simple method.

Brief Description of Drawings

FIGS. 1A to 1D are front views of artificial cartilage implants according to an embodiment, and FIG. 1E is a cross-sectional view of the artificial cartilage implant illustrated in FIG. 1A;
FIG. 2A is a schematic diagram for describing a method of performing a Push-Out test in an example, and FIG. 2B is a graph showing the test results of the Push-Out test; and
FIG. 3 is a graph showing the results of the fracture stress test in the example.

Description of Embodiments

In the present specification, a numerical range represented by using a term "to" or "-" means a range including numerical values described before and after the term as lower and upper limits. In the numerically limited range described in the present specification, the upper limit value or the lower limit value described in a certain numerically limited range may be replaced with the upper limit value or the lower limit value of the numerically limited range described in another stepwise manner.

1. Artificial Cartilage Implant

An artificial cartilage implant is a member that functions as a substitute for cartilage by being implanted in a living body. Hereinafter, the present invention will be described with reference to an embodiment. However, the artificial cartilage implant of the present invention is not limited to the above-described embodiment.

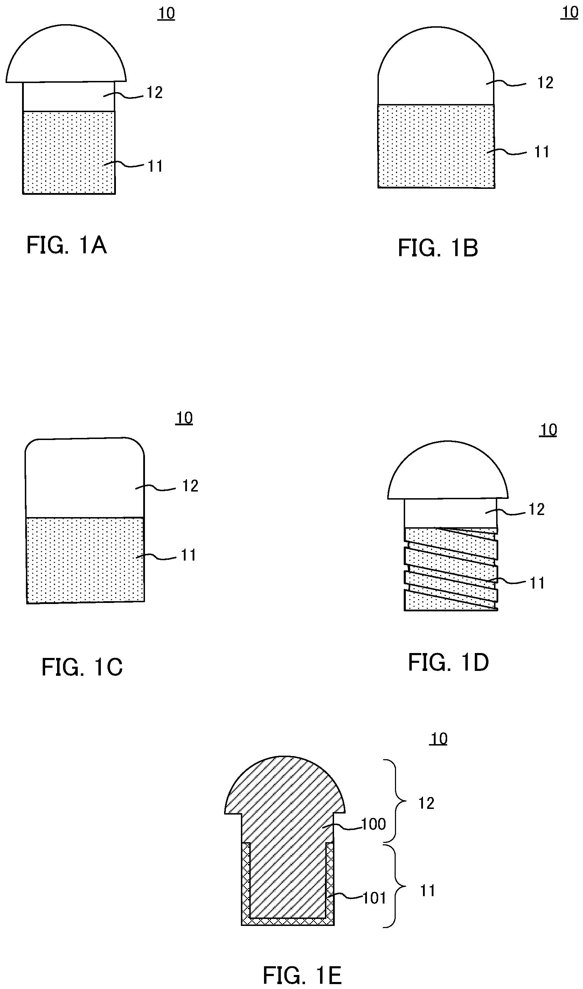

FIG. 1A is a front view of an artificial cartilage implant according to an embodiment. As illustrated in FIG. 1A, artificial cartilage implant 10 of the present embodiment has a structure such that a spherical crown having a diameter larger than that of a cylinder is joined to an end surface of the cylinder. The shape of artificial cartilage implant 10 is not limited to the shape described above, and may be, for example, a structure such that the corners at one end of a cylinder is radius chamfered, as illustrated in FIGS. 1B and 1C. Further, for example, as illustrated in FIG. 1D, the artificial cartilage implant may have a structure such that a screw groove is formed in a part thereof (bone implant section 11 in FIG. 1D). Further, the shape of artificial cartilage implant 10 is not limited to a columnar shape, and may be various shapes according to the shape of a portion of a living body in which artificial cartilage implant 10 is used.
As illustrated in FIGS. 1A to 1D, artificial cartilage implant 10 in any shape includes a section to be implanted into a bone (herein, simply referred to as "bone implant section") 11 and artificial cartilage section 12. The ratio of the height of bone implant section 11 to the height of artificial cartilage section 12 in artificial cartilage implant 10 is appropriately selected according to the shape of the bone into which artificial cartilage implant 10 is to be embedded or the shape of the cartilage that artificial cartilage section 12 to replace.
Bone implant section 11 is a portion for embedding in a bone, and in the present embodiment, as illustrated in FIG. 1A, is a cylindrical portion. Bone implant section 11 includes a polymer gel and hydroxyapatite particles dispersed at least in the vicinity of the surface of the polymer gel. In the present embodiment, as illustrated in the cross-sectional view of FIG. 1E (cross-sectional view of the artificial cartilage implant illustrated in FIG. 1A), hydroxyapatite particles 101 are dispersed in the outer peripheral surface of cylindrical polymer gel 100 of bone implant section 11 and in the vicinity of the bottom surface on one side. In bone implant section 11, however, hydroxyapatite particles may be dispersed throughout the entire polymer gel.
The amount of hydroxyapatite particles in the polymer gel of bone implant section 11 is not particularly limited as long as it is possible to form a dense structure with inorganic components in the body and to enhance the joining strength with a bone when bone implant section 11 is embedded in the bone. When the cross section of the polymer gel of bone implant section 11 is observed, it is preferable that hydroxyapatite particles are distributed in a region from the surface to a depth of 1000 $\mu$m, and the content of the hydroxyapatite particles at a depth of 1000 $\mu$m from the surface is 0.1% by weight or more and 80% by weight or less. It is more preferable that the lower limit value of the content is 0.5% by weight. Hydroxyapatite particles may be distributed throughout the entire region from the surface to a depth of 1000 $\mu$m; however, for example, the hydroxyapatite particles may be distributed in greater numbers on the surface side, and the distribution of hydroxyapatite particles may become small or almost nonexistent on the deeper side. That is, in bone implant section 11, the total amount of hydroxyapatite particles included in the region from the surface to a depth of 1000 μm may be 1% by weight or more based on the total amount of bone implant section 11 in the region from the surface to a depth of 1000 μm. When the amount of hydroxyapatite particles in the vicinity of the surface of the polymer gel in bone implant section 11 is within the above range, the joining strength between bone implant section 11 and the bone is more likely to become high when artificial cartilage implant 10 is embedded in the bone. In the present specification, the "cross section of a polymer gel of bone implant section 11" refers to a cross section obtained by cutting the polymer gel substantially perpendicularly to the upper surface (the surface on the artificial cartilage section 12 side) of bone implant section 11 such that the cutting passes through the center of bone implant section 11. In the present embodiment, the cross section is obtained by cutting a cylindrical polymer gel along its central axis. Further, in the present specification, "depth from the surface" refers to the distance from the surface in the direction of the central axis.

The content of hydroxyapatite particles in a region from the surface to a depth of 1000 μm in bone implant section 11 is calculated as follows by using a thermogravimetric analyzing apparatus. The polymer gel of bone implant section 11, including hydroxyapatite particles and water, is taken, and is heated at a temperature rising rate of 10°C per minute in a range of 30°C to 600°C. The water content is determined by the total weight change from 30°C to a temperature at which the differential thermal analysis value indicates 0 (approximately 150 to 200°C). The hydroxyapatite particle fraction is determined from the final residue at 600°C, and the polymer gel fraction is determined by the difference between the total weight and the sum of the weights of water and the hydroxyapatite particles. The thickness of the polymer gel in the depth direction from the surface can be quantified by observation of a cross section using a field emission transmission electron microscope (FE-TEM) or the like. The content (C) in percent by weight of the hydroxyapatite particles included up to a depth of 1000 μm is calculated from the calculation formula (C) = (B) ÷ {(A) × (D) + (B)}, where the volume fraction (A) up to a depth of 1000 μm from the surface of the polymer gel is determined by calculation, and the total amount (B) of the hydroxyapatite particles and the content (D) of the polymer gel component are determined by the thermogravimetric analysis. For example, in a case where the polymer gel has a cylindrical shape, when the radius of the cylinder is R1 (μm) and the height is L1 (μm), the volume fraction of the region from the surface to a depth of 1000 μm is calculated as (A) = { $(\pi \times (R1)^2 \times L1) - (\pi \times (R1 - 1000)^2 \times L1)$ } ÷ $(\pi \times (R1)^2 \times L1)$. In a case where R1 is smaller than 1000 μm, the content of the hydroxyapatite particles is calculated as (A) = 1. Further, in a case where the hydroxyapatite particles are present in a range deeper than 1000 μm from the surface, the volume fraction (E) of the hydroxyapatite particles from the surface to

1000 μm is also determined by calculation, and (C) is calculated by the calculation formula of (C) = {(B) × (E)} ÷ {(A) × (D) + (B) × (E)}.

On the other hand, artificial cartilage section 12 is a portion to be used as a substitute for cartilage. In the present embodiment, as illustrated in FIG. 1A, artificial cartilage section 12 is composed of a cylindrical portion and a spherical crown portion joined to an end surface of the cylinder. Artificial cartilage section 12 is mainly composed of polymer gel, and the polymer gel of artificial cartilage section 12 is formed integrally with the polymer gel of bone implant section 11.

In artificial cartilage section 12, the amount of hydroxyapatite in the vicinity of the surface of the polymer gel is less than a certain value. Specifically, when the cross section of the polymer gel of artificial cartilage section 12 is observed, no hydroxyapatite particles are distributed in a region from the surface to a depth of 1000 μm, or in a case where hydroxyapatite particles are distributed in the region, the content of the hydroxyapatite particles at a depth of 1000 μm from the surface is less than 0.1% by weight. The content of hydroxyapatite particles in the polymer gel of artificial cartilage section 12 is preferably 0.05% by weight or less. When the amount of hydroxyapatite particles in the vicinity of the surface of the polymer gel of artificial cartilage section 12 is within the above range, the strength and elasticity of artificial cartilage section 12 are less likely to change over time when artificial cartilage implant 10 is embedded in a bone. In the present specification, the "cross section of a polymer gel of artificial cartilage section 12" refers to a cross section obtained by cutting the polymer gel substantially perpendicularly to the bottom surface (the surface on bone implant section 11 side) of artificial cartilage section 12 such that the cutting passes through the center of artificial cartilage section 12. In the present embodiment, the cross section of the polymer gel with the above-described shape is cut through the central axis. Further, the content of hydroxyapatite particles in the polymer gel of artificial cartilage section 12 can be calculated by taking the polymer gel of artificial cartilage section 12 out and subjecting the sample to the same method as in the calculation of the content of hydroxyapatite particles in the polymer gel of bone implant section 11.

The polymer gel included in the above-described artificial cartilage implant 10 (bone implant section 11 and artificial cartilage section 12) may be any polymer gel that is capable of dispersing hydroxyapatite particles therein and has biocompatibility. Examples of the polymer gel include polymer gels having a hydroxyl group, an amide group, an ester group, and the like. Specific examples of materials for the polymer gel include polyvinyl alcohol; materials having an amide group such as poly(N-methylacrylamide), poly(N-ethylacrylamide), and poly(acrylamide); materials having an ester group such as poly(methoxyethyl acrylate), poly(ethoxyethyl acrylate), poly(methoxyethyl methacrylate), and poly(ethoxyethyl methacrylate); and the like.

On the other hand, as described in the manufacturing method of artificial cartilage implant 10 below, an acidic solution is used in the case of dispersing (forming) hydroxyapatite particles in a polymer gel. Accordingly, it is preferable that the polymer gel is less likely to decrease in elastic modulus and strength and further is less likely to decrease in swelling or shrinking even when the polymer gel is brought into contact with an acidic solution. Accordingly, a polymer gel including a first mesh structure and a second mesh structure that is entangled with the first mesh structure, namely a double mesh structure (hereinafter, also referred to as a "double network gel"), is particularly preferable. Such a double network gel undergoes little change in elastic modulus or strength even when immersed in an acidic solution, and is also less likely to swell or shrink.

The double network gel can be produced as follows. However, the production method of a double network gel is not limited to the following method. First, a first polymerization solution including a first monomer, a polymerization initiator, and a crosslinking agent is prepared. Then, the first monomer is polymerized and crosslinked to form the first mesh structure. Subsequently, a second polymerization solution including a second monomer, a polymerization initiator, and a second crosslinking agent is prepared, and the first mesh structure is immersed in the second polymerization solution to diffuse and permeate the second polymerization solution into the first mesh structure. Subsequently, the first mesh structure is taken out, and the polymerization and crosslinking of the second monomer are performed. Thus, a double network gel in which the first mesh structure and the second mesh structure are entangled with each other is obtained. The polymerization and crosslinking method of the first monomer and the second monomer is not particularly limited, and can be performed, for example, by heating or light irradiation.

Examples of the above first monomer and second monomer include monomers having a charge, such as 2-acrylamido-2-methylpropanesulfonic acid, acrylic acid, methacrylic acid, and the like; and electrically neutral monomers such as acrylamide, N',N-dimethylacrylamide, N-isopropylacrylamide, vinylpyridine, styrene, methyl methacrylate, fluorine-containing unsaturated monomers (for example, trifluoroethyl acrylate and the like), hydroxyethyl acrylate, vinyl acetate, and the like. As the first monomer and the second monomer, only one of these may be used, or two or more of these may be used. The first monomer and the second monomer may be of the same type or composition, or they may be of different types or compositions.

Further, examples of the first crosslinking agent and the second crosslinking agent include the following: crosslinking agents not including a degradable bond, such as divinylbenzene, divinylpyridine, divinylbiphenyl, and divinylsulfone; and crosslinking agents including a degradable bond, such as N,N'-methylenebisacrylamide and ethylene glycol dimethacrylate. **In** the present specification, the term "degradable bond" refers to a bond that is easily hydrolyzed under an acidic condition, and examples thereof include ester bond, amide bond, thioester bond, and the like. As the first crosslinking agent and the second crosslinking agent, only one of the crosslinking agents may be used, or two or more of these may be used, independently. However, it is preferable that 50 mol% or more of the first crosslinking agent is a crosslinking agent not including a degradable bond. Similarly, it is preferable that 50 mol% or more of the second crosslinking agent is a crosslinking agent not including a degradable bond. The proportion of the crosslinking agent not including a degradable bond is more preferably 60 mol% or more, and further preferably 75 mol% or more, independently. By using a large amount of each of the first and second crosslinking agents that does not contain a decomposable bond, the polymer gel becomes less likely to decompose when forming hydroxyapatite particles. The first crosslinking agent and the second crosslinking agent may be the same in type and composition. The amount (mol) of the first crosslinking agent used is preferably 0. 1 to 50 mol% with respect to the amount (mol) of the first monomer used. On the other hand, the amount (mol) of the second crosslinking agent is preferably 0.001 to 20 mol% with respect to the amount (mol) of the second monomer used. Thus, a double network gel in which the hardness of the first cross-linked structural body is high and the elasticity of the second cross-linked structural body is high is obtained.

Further, the polymerization initiator used in the first polymerization solution or the second polymerization solution is appropriately selected according to the type of monomer and the polymerization method. Examples of the polymerization initiator include watersoluble thermal catalysts such as potassium persulfate, redox initiators such as potassium persulfate-sodium thiosulfate, thermal polymerization initiators such as azobisisobutyronitrile (AIBN) and benzoyl peroxide (BPO), and photopolymerization initiators such as 2-oxoglutaric acid, benzophenone, and hydrogen peroxide.

The double network gel preferably has an initial elastic modulus ratio X defined by the Equation (1) below of 50% or more. When the initial elastic modulus ratio is 50% or more, after artificial cartilage implant 10 is embedded in a bone, artificial cartilage section 12 has an appropriate elasticity and is more likely to function as a substitute for cartilage.

$$X = (E2/E1) \times 100 \qquad \ldots \text{Equation (1)}$$

In the above Equation (1), E2 represents the elastic modulus of the polymer gel after being subjected to an immersion treatment in a hydrochloric acid aqueous solution with a concentration of 0.05 mol/L at 60°C for 72 hours, and E1 represents the elastic modulus of the polymer gel before the immersion treatment.

Further, when M1 (mol%) is defined as the structural amount derived from the first monomer in the above double network gel and M2 (mol%) is defined as the structural amount derived from the second monomer, it is preferable that M1:M2 is 1:2 to 1:100, more preferably 1:3 to 1:50, and even more preferably 1:3 to 1:30. Such ratio is more likely to further increase the mechanical strength of the double network gel.

In addition, the water content of the double network gel is preferably 10 to 99.9%. Further, the compressive fracture stress of the double network gel is preferably 1 to 100 MPa. The compressive fracture stress is the stress at which a double network gel fractures when the double network gel is sandwiched between two flat plates and compressed at a predetermined rate (a rate at which the thickness of the sample is compressed by 10% per minute). On the other hand, the tensile fracture force is preferably 0.1 to 100 MPa. Further, the fracture energy is preferably 100 J/m$^2$ or more. The tensile stress and the fracture energy are measured by preparing a dumbbell-type hydrogel (ISO-37-4 standard size: length 11 mm, width 2.0 mm, and thickness 3.0 mm) and using a tensile tester (Tensilon RTC-1310A, manufactured by Orientec Co., Ltd.) at a test speed of 100 mm/min.

A hydroxyapatite particle refers to a particle of a calcium phosphate compound represented by $Ca_{10}(PO_4)_6(OH)_2$. The shape of the hydroxyapatite particle is usually spherical with protrusions. The hydroxyapatite particles may be included as primary particles in the polymer gel (mainly bone implant section 11) or may be included as an aggregate. The average particle diameter of the hydroxyapatite particles in the above polymer gel is preferably about 1 to 100,000 nm, and more preferably 10 to 10,000 nm. When the average particle diameter of the hydroxyapatite particles is within the range, the particles are more likely to react with inorganic substances in the living body to form a dense structure. The average particle diameter of the above-described hydroxyapatite particles is a value calculated as an average value of the particle diameters of 100 hydroxyapatite particles in a cross section of artificial cartilage implant 10 measured with a field emission transmission electron microscope (FE-TEM) or the like.

Artificial cartilage implant 10 of the present embodiment may further include components other than the above-described polymer gel and hydroxyapatite particles, as long as the effects and purposes of the present invention are not impaired. Artificial cartilage implant 10 may also include regions other than bone implant section 11 and artificial cartilage section 12 as long as the effects and purposes of the present invention are not impaired.

(Effect)

An artificial cartilage implant of the present invention includes a bone implant section and an artificial cartilage section. Further, the artificial cartilage implant includes hydroxyapatite particles in the vicinity of the surface of the polymer gel of the bone implant section; therefore, the joining strength between the bone implant section and a bone is more likely to be improved when the artificial cartilage implant is embedded in the bone. More specifically, the hydroxyapatite particles on the surface of the polymer gel of the bone implant section or inside the bone implant section react with inorganic components in the living body to form a dense structure. Further, as the structure integrates with the bone, the joining strength between the artificial cartilage implant and the bone becomes very high. On the other hand, the artificial cartilage section of the artificial cartilage implant of the present invention has a very small amount of hydroxyapatite particles present in the vicinity of the surface. For this reason, the artificial cartilage section is less likely to become ceramic-like, which would be caused by the deposition of inorganic components in the body. As a result, the strength and elasticity are less likely to change over time. Accordingly, the artificial cartilage section can maintain a low elastic modulus over a long period of time and can easily function as a substitute for cartilage.

2. Manufacturing Method of Artificial Cartilage Implant

The artificial cartilage implant including the bone implant section and the artificial cartilage section described above can be manufactured, for example, by the following two methods. However, the manufacturing method of an artificial cartilage implant is not limited to these methods.

The first manufacturing method includes the following steps: preparing a polymer gel (polymer gel preparation step); and forming hydroxyapatite particles in the vicinity of a surface of a region (in the polymer gel) corresponding to the bone implant section by sequentially bringing an alkali metal phosphate aqueous solution and a calcium salt aqueous solution into contact with the region of the polymer gel corresponding to the bone implant section while protecting a region (in the polymer gel) corresponding to the artificial cartilage section (hydroxyapatite particle formation step).

In the polymer gel preparation step in the first manufacturing method, polymer gel having a desired shape may be prepared. For example, a commercially available polymer gel may be used as it is. Further, a commercially available polymer gel may be processed or molded into a desired shape. Further, a double network gel having a desired shape may be prepared based on the production method of the double network gel described above. During the production of a double network gel, the polymer gel can be molded into a desired shape by using a mold when the first monomer is polymerized and when the second monomer is polymerized.

Further, in the hydroxyapatite particle formation step in the first manufacturing method, an alkali metal phosphate aqueous solution and a calcium salt aqueous solution are sequentially brought into contact with the region (in the polymer gel) corresponding to the bone implant section while protecting the region (in the polymer gel) corresponding to the artificial cartilage section. The method for protecting the region corresponding to the artificial cartilage section is not particularly limited, as long as the region corresponding to the artificial cartilage section substantially does not contact an alkaline metal phosphate aqueous solution or a calcium salt aqueous solution. For example, the region corresponding to the artificial cartilage section may be protected by bringing the alkali metal phosphate aqueous solution and the calcium salt aqueous solution into contact with only the polymer gel in the region corresponding to the bone implant section, while preventing the polymer gel in the region corresponding to the artificial cartilage section from coming into contact with the alkali metal phosphate aqueous solution and the calcium salt aqueous solution. Further, the region of the polymer gel corresponding to the artificial cartilage section may be temporarily covered and protected with a member such as masking tape.

Specifically, only the region corresponding to the bone implant section may be sequentially immersed in an alkali metal phosphate aqueous solution and a calcium salt aqueous solution to form hydroxyapatite particles in the region corresponding to the bone implant section, or only the region corresponding to the bone implant section may be sequentially coated with an alkali metal phosphate aqueous solution and a calcium salt aqueous solution to form hydroxyapatite particles in the region corresponding to the bone implant section. Further, the region corresponding to the artificial cartilage section may be protected with a masking tape or the like, and the entire polymer gel may be sequentially immersed in an alkali metal phosphate aqueous solution and a calcium salt aqueous solution.

The alkali metal phosphate aqueous solution used in the present step may be any aqueous solution of an alkali metal phosphate, and examples of the alkali metal phosphate include disodium hydrogen phosphate, dipotassium hydrogen phosphate, and the like. In addition, the calcium salt aqueous solution may be any aqueous solution of a calcium salt, and examples of the calcium salt include calcium chloride, calcium carbonate, and the like.

The order in which the polymer gel (region corresponding to the bone implant section) is brought into contact with the alkali metal phosphate aqueous solution and the calcium salt aqueous solution is not particularly limited, and either may be first. Further, the number of times of bringing these solutions into contact with the polymer gel is not particularly limited, either, and may be, for example, once for each solution or twice or more for each solution. By increasing the number of contacts, it is possible to increase the amount of hydroxyapatite particles included in the vicinity of the surface of the polymer gel of the bone implant section. Further, the contact time between the polymer gel and each solution is appropriately selected according to the concentration of each solution, the size of the polymer gel, and the desired amount of hydroxyapatite particles. After the contact between the polymer gel and each solution, the polymer gel may be washed with pure water or the like as necessary.

On the other hand, the second manufacturing method includes the following steps: preparing polymer gel (polymer gel preparation step); forming the hydroxyapatite particles in the vicinity of a surface of the polymer gel by sequentially bringing an alkali metal phosphate aqueous solution and a calcium salt aqueous solution into contact with the polymer gel (hydroxyapatite particle formation step); and removing the hydroxyapatite particles in a region (of the polymer gel after forming the hydroxyapatite particles) corresponding to the artificial cartilage section by bringing an acidic solution into contact with the region corresponding to the artificial cartilage section while protecting a region corresponding to the bone implant section (hydroxyapatite particle removal step). The polymer gel preparation step is the same as the polymer gel preparation step in the first manufacturing method described above, and thus, a detailed description thereof will be omitted here.

In the hydroxyapatite particle formation step in the second manufacturing method, the polymer gel is sequentially brought into contact with an alkali metal phosphate aqueous solution and a calcium salt aqueous solution. At this time, the entire polymer gel may be brought into contact with an alkali metal phosphate aqueous solution and a calcium salt aqueous solution without distinguishing between the bone implant section and the artificial cartilage section. However, as in the first manufacturing method, only the region corresponding to the bone implant section may be brought into contact with the alkali metal phosphate aqueous solution and the calcium salt aqueous solution while protecting the region corresponding to the bone implant section. The types of the alkali metal phosphate aqueous solution and the calcium salt aqueous solution, the method of bringing the polymer gel into contact with each solution, the contact time, and the like are the same as those in the first manufacturing method.

Further, in the hydroxyapatite particle removal step in the second manufacturing method, the hydroxyapatite particles in the region (of the polymer gel after the hydroxyapatite particle formation step) corresponding to the artificial cartilage section are removed by bringing an acidic solution into contact with the region corresponding to the artificial cartilage section while protecting the region corresponding to the bone implant section. The method for protecting the bone implant section is the same as the method for protecting the artificial cartilage section in the hydroxyapatite particle formation step of the first manufacturing method. For example, the following may be used: a method in which only the polymer gel in the region corresponding to the artificial cartilage section is immersed in the acidic solution such that the acidic solution does not contact the region corresponding to the bone implant section, or a method in which the acidic solution is applied only to the polymer gel in the region corresponding to the artificial cartilage section. Further, the region corresponding to the bone implant section may be covered with a protective material such as masking tape, and the entire polymer gel may be brought into contact with an acidic solution.

The type of acidic solution used in the present step is not particularly limited, and examples thereof include aqueous solutions of hydrochloric acid, phosphoric acid, sulfuric acid, nitric acid, and the like. The time for treating the region corresponding to the artificial cartilage section with an acidic solution is not particularly limited, and is appropriately selected according to the amount of hydroxyapatite particles formed in the hydroxyapatite particle formation step. After the contact between the polymer gel and the acidic solution, the polymer gel may be washed with pure water or the like as necessary.

(Effect)

The manufacturing method of the artificial cartilage implant described above can produce, by a simple method, an artificial cartilage implant that can be used stably over a long period of time with little change even when used in a living body.

Examples

Hereinafter, the present invention will be described in more detail with reference to Examples. The scope of the present invention is not limited to these examples, and modifications to the embodiments are possible without departing from the spirit of the present invention.

1. Production of Artificial Cartilage Implant

(1) Sample 1

(Polymer Gel Preparation Step)

To a mixed solvent of pure water and dimethyl sulfoxide (DMSO), 1 mol/L of a first monomer (2-acrylamido-2-methylpropanesulfonic acid (AMPS)), 0.04 mol/L of a crosslinking agent (divinylbenzene (DVB)), and 0.01 mol/L of an initiator (2-oxoglutaric acid) were added to obtain a first polymerization solution. The obtained first polymerization solution was deoxygenated with nitrogen gas and poured into a glass mold (having a shape such that a bottom surface of a hemisphere with a radius of 4 mm and a height of 2 mm is connected to one end surface of a cylinder with a radius of 3 mm and a height of 4 mm). Subsequently, the first polymerization solution was irradiated with ultraviolet light from a UV lamp (22 W, 0.34 A) with a wavelength of 365 nm for 8 hours at room temperature, and the first monomer was polymerized and crosslinked to produce an AMPS gel (first mesh structure).

Subsequently, 2 mol/L of a second monomer (N',N-dimethylacrylamide (DMAAm)), 0.3 mol/L of a crosslinking agent (divinylbenzene), and 0.005 mol/L of an initiator (2-oxoglutaric acid) were dissolved in a mixed solvent of water and DMSO to obtain a second polymerization solution. Subsequently, the above-described AMPS gel (first mesh structure) was immersed in the second polymerization solution for 24 hours, and the second polymerization solution was diffused and penetrated into the AMPS gel. Subsequently, the AMPS gel was taken out from the second polymerization solution and installed in the same glass mold as described above. Then, the AMPS gel was irradiated with ultraviolet light from a UV lamp (22 W, 0.34 A) with a wavelength of 365 nm for 8 hours at room temperature. Thus, the DMAAm monomer diffused in the AMPS gel was polymerized and crosslinked to obtain a double network gel (DN gel) in which a first mesh structure and a second mesh structure having different characteristics were entangled with each other.

(Hydroxyapatite Particle Formation Step)

The region of the DN gel produced above corresponding to the artificial cartilage section was masked with masking tape. Subsequently, the DN gel was immersed overnight in a tris(hydroxymethyl)aminomethane aqueous solution (1.0 mol/L) adjusted to pH 9.0. The sample was then immersed in pure water for 10 seconds to remove the excess tris(hydroxymethyl)aminomethane aqueous solution. The excess water was wiped off with a cloth or the like. Subsequently, the DN gel was immersed in a calcium chloride aqueous solution (0.5 mol/L) for 2 minutes, then immersed again in pure water for 10 seconds, and the excess water was wiped off. Subsequently, the sample was immersed in a dipotassium hydrogen phosphate aqueous solution (0.3 mol/L) for 2 minutes. The process from immersion in the calcium chloride aqueous solution to immersion in the dipotassium hydrogen phosphate aqueous solution was defined as one cycle, and five cycles were performed. The DN gel that has been subjected to the immersion treatment was immersed overnight in a calcium chloride aqueous solution adjusted to pH 11 at 37°C, and then the masking tape was removed. The above processes obtained an artificial cartilage implant in which hydroxyapatite particles are selectively dispersed in the vicinity of the surface of polymer gel (DN gel) in the bone implant section.

(Confirmation of Hydroxyapatite Particles)

The artificial cartilage implant produced by the above method was cut along the central axis, and the cut surface was observed with a field emission transmission electron microscope (FE-TEM). In the cross-sectional observation, the hydroxyapatite particles were not distributed in a region from the arc surface of the polymer gel of the artificial cartilage section (portion with a shape such that a bottom surface of a hemisphere with a radius of 4 mm and a height of 2 mm is connected) to the inside by 1000 $\mu$m. On the other hand, hydroxyapatite particles are mainly distributed in a region from the arc surface of the polymer gel of the bone implant section (cylindrical portion with a radius of 3 mm and a height of 4 mm) to the inside by 500 $\mu$m, and the content of the hydroxyapatite particles in the bone implant section was 33% by weight. Accordingly, the content of hydroxyapatite particles in a region from the surface of the polymer gel of the bone implant section to 1000 $\mu$m is 36% by weight or more, since the volume fraction (A) in the region from the surface to 1000 $\mu$m is 0.89. That is, in the artificial cartilage implant, the amount of hydroxyapatite particles in the artificial cartilage section was very small, while the amount of hydroxyapatite particles in the bone implant section was sufficiently large.

(2) Sample 2

(Polymer Gel Preparation Step)

In the same manner as in Sample 1, a double network gel (DN gel) was prepared.

(Hydroxyapatite Particle Formation Step)

Except for not masking the region corresponding to the artificial cartilage section with masking tape, the same procedure as in Sample 1 was performed, including five cycles of immersion in a calcium chloride aqueous solution and immersion in a dipotassium hydrogen phosphate aqueous solution, resulting in the formation of hydroxyapatite particles in the vicinity of the entire surface of the DN gel.

(Hydroxyapatite Particle Removal Step)

Only the region of the DN gel corresponding to the artificial cartilage section was immersed in a 10 mass% aqueous hydrochloric acid aqueous solution, and the hydroxyapatite particles in the region corresponding to the artificial cartilage section were removed. Thus, an artificial cartilage implant in which hydroxyapatite particles are selectively dispersed in the vicinity of the surface of the polymer gel (DN gel) in the bone implant section was obtained.

(Confirmation of Hydroxyapatite Particles)

The artificial cartilage implant produced by the above method was cut along the central axis, and the cut surface was observed with a field emission transmission electron microscope (FE-TEM). In the cross-sectional observation, hydroxyapatite particles were not distributed in a region from the surface of the polymer gel of the artificial cartilage section to the inside by 1000 $\mu$m. On the other hand, hydroxyapatite particles are mainly distributed in a region from the surface of the polymer gel of the bone implant section to the inside by 500 $\mu$m, and the content of the hydroxyapatite particles in the bone implant section is 33% by weight or more. Accordingly, it can be said that the content of hydroxyapatite particles in a region from the surface of the polymer gel of the bone implant section to 1000 $\mu$m is 36% by weight or more. That is, in the artificial cartilage implant, the amount of hydroxyapatite particles in the artificial cartilage section was very small, while the amount of hydroxyapatite particles in the bone implant section was sufficiently large.

(3) Sample 3

(Production of Artificial Cartilage Implant)

An artificial cartilage implant was produced in the same manner as in Sample 2, except that the hydroxyapatite particle removal step was not performed.

(Confirmation of Hydroxyapatite Particles)

The artificial cartilage implant produced by the above method was cut along the central axis, and the cut surface was observed with a field emission transmission electron microscope (FE-TEM). **In** the cross-sectional observation, hydroxyapatite particles were mainly distributed in regions from the surfaces of the polymer gel of the artificial cartilage section and the bone implant section to the inside by 500 $\mu$m, and the content of the hydroxyapatite particles in the artificial cartilage section and the bone implant section, that is, in the artificial cartilage implant, was 33% by weight. Accordingly, it can be said that the content of hydroxyapatite particles in regions from the surfaces of the polymer gel of the artificial cartilage section and the bone implant section to the inside by 1000 $\mu$m is 36% by weight or more. That is, in the artificial cartilage implant, a large amount of hydroxyapatite particles was dispersed in the vicinity of the surfaces of the artificial cartilage section and the bone implant section.

2. Evaluation of Knee Mobility Score

A cartilage defect was created in rabbit femoral trochleae (patellar facing surfaces), and the bone implant sections of the artificial cartilage implants produced in Sample 1 and Sample 2 were embedded. Further, the mobility of the knee was monitored over a period of 12 months, and there were no issues.

3. Push-Out Test (Evaluation of Bonding Property of Bone Implant Section with Living Body)

In the same manner as in Sample 1, an artificial cartilage implant (Sample 4) including hydroxyapatite particles in the vicinity of the surface (approximately 150 $\mu$m from the surface) of the polymer gel of the bone implant section was produced. Further, the number of times of immersion in the calcium chloride aqueous solution and immersion in the dipotassium hydrogen phosphate aqueous solution was changed to produce an artificial cartilage implant (Sample 5) including hydroxyapatite particles in the vicinity of the surface (approximately 300 μm from the surface) of the polymer gel of the bone implant section. Further, an artificial cartilage implant (Sample 6) consisting only of polymer gel was produced without performing immersion in a calcium chloride aqueous solution and immersion in a dipotassium hydrogen phosphate aqueous solution.

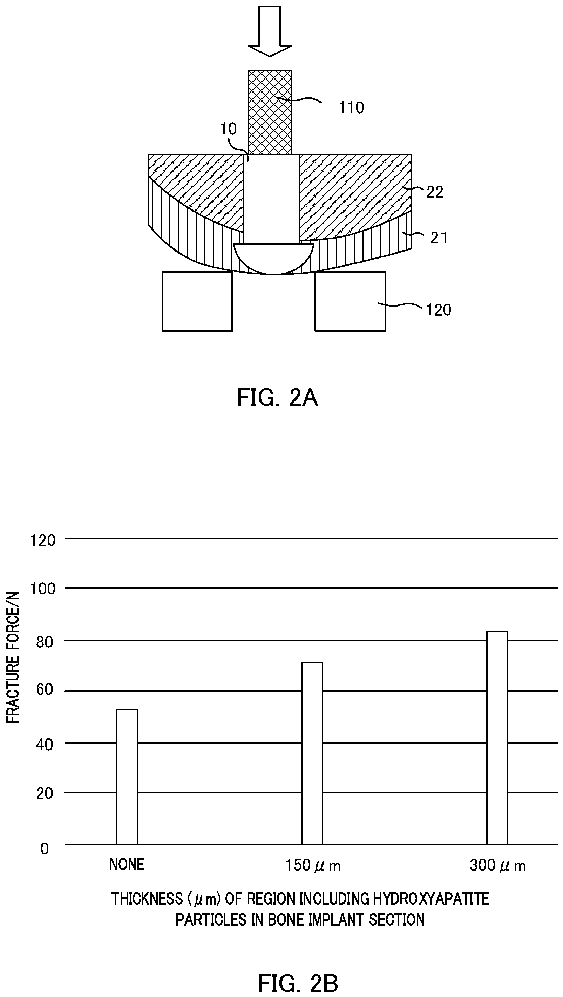

Each of these artificial cartilage implants (Samples 4 to 6) was implanted in a rabbit femoral trochlea (patellar facing surface) in the same manner as in the knee mobility score measurement. As shown in FIG. 2A, each femur (cartilage 21 and bone 22) eight weeks after the implantation of the artificial cartilage implant was fixed to lower jig 120 made of metal, and artificial cartilage implant 10 was pushed out from the bone 22 side to the cartilage 21 side (direction indicated by the arrow in FIG. 2A) with indenter 110. The maximum value of the force required to push out artificial cartilage implant 10 was used as the fracture force (N). FIG. 2B shows the results.

As illustrated in FIG. 2B, compared with the fracture force in a case where the bone implant section does not include hydroxyapatite particles (0 μm (Sample 6)), the fracture force was significantly large in a case where the bone implant section includes hydroxyapatite particles (150 μm (Sample 4) and 300 μm (Sample 5)), and it is clear that the bone and the bone implant section are firmly bonded to each other.

4. Fracture Stress Test (Evaluation of Time-Dependent Change in Fracture Stress of Artificial Cartilage Section in Living Body)

An artificial cartilage implant (Sample 7) including hydroxyapatite particles in the vicinity of the entire surface of the polymer gel was produced by the same method as in Sample 2 (except that the hydroxyapatite particle removal step was not performed). In the hydroxyapatite particle formation step, the immersion in the calcium chloride aqueous solution and the immersion in the dipotassium hydrogen phosphate aqueous solution were each performed 10 times.

Similarly, an artificial cartilage implant (Sample 8) including hydroxyapatite particles in the vicinity of the entire surface of a polymer gel was produced. In the hydroxyapatite particle formation step, the immersion in the calcium chloride aqueous solution and the immersion in the dipotassium hydrogen phosphate aqueous solution were each performed 15 times.

Further, an artificial cartilage implant (Sample 3 described above) consisting only of polymer gel was produced without performing immersion in a calcium chloride aqueous solution and immersion in a dipotassium hydrogen phosphate aqueous solution.

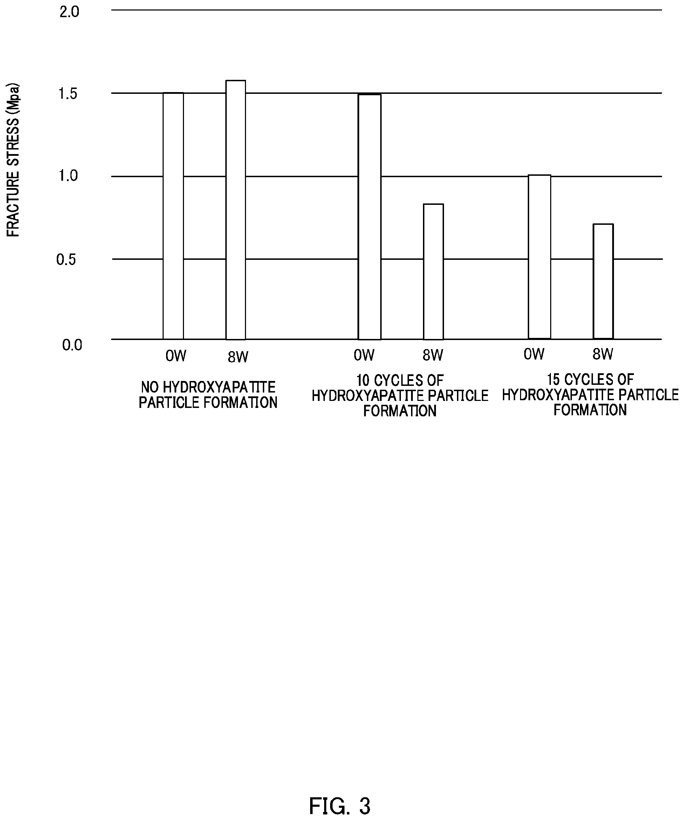

These artificial cartilage implants (Samples 3, 7, and 8) were immersed in a simulated body fluid. The fracture stress of the polymer gel before and after 8 weeks of immersion was measured using a Tensilon universal testing machine RTC-1310A (manufactured by Orientec Co., Ltd.). The results at this time are illustrated in FIG. 3. As shown in FIG. 3, in a case where the artificial cartilage implant does not include hydroxyapatite particles, the fracture stress did not decrease. On the other hand, in the case of including hydroxyapatite particles, the fracture stress was significantly reduced.

5. Summary

From the results of the above Push-Out test, it can be said that, in the bone implant section of the artificial cartilage implant, when the surface vicinity of the polymer gel includes hydroxyapatite particles, the joining strength is significantly increased when the artificial cartilage implant is embedded in a living body and excellent effects can be obtained. On the other hand, the results of the above-described fracture stress test indicate that, in the artificial cartilage section, the fracture stress does not decrease and the artificial cartilage section can be used stably over a long period of time when the surface vicinity of the polymer gel does not include hydroxyapatite particles. That is, by forming an artificial cartilage implant in which a bone implant section includes a large amount of hydroxyapatite particles and an artificial cartilage section includes a small amount of hydroxyapatite particles, the artificial cartilage implant can be fixed to the bone and can be used stably for a long period of time.

The present application is entitled to and claims the benefit of Japanese Patent Application No. 2022-094413, filed on June 10, 2022, the disclosure of which including the specification and drawings is incorporated herein by reference in its entirety.

Industrial Applicability

The artificial cartilage implant of the present invention can be used stably over a long period of time with little change even when used in a living body. Accordingly, the present invention is very useful in the medical field.

Reference Signs List

    10 Artificial cartilage implant
    11 Bone implant section
    12 Artificial cartilage section
    21 Cartilage
    22 Bone
    100 Polymer gel
    101 Hydroxyapatite particle
    110 Indenter

120 Lower jig

**Claims**

1. An artificial cartilage implant, comprising:

   a bone implant section including a polymer gel and hydroxyapatite particles dispersed at least in a vicinity of a surface of the polymer gel; and an artificial cartilage section including a polymer gel, wherein when a cross section of the polymer gel of the artificial cartilage section is observed, no hydroxyapatite particle is distributed in a region from a surface to a depth of 1000 $\mu$m, or in a case where hydroxyapatite particles are distributed in the region, a content of the hydroxyapatite particles at the depth of 1000 $\mu$m from the surface is less than 0.1% by weight.

2. The artificial cartilage implant according to claim 1, wherein:
   when a cross section of the polymer gel of the bone implant section is observed, the hydroxyapatite particles are distributed in a region from a surface to a depth of 1000 $\mu$m, and a content of the hydroxyapatite particles at the depth of 1000 $\mu$m from the surface is 0.1% by weight to 80% by weight.

3. The artificial cartilage implant according to claim 1, wherein:
   the polymer gel of the bone implant section and the polymer gel of the artificial cartilage section are integrated with each other.

4. The artificial cartilage implant according to claim 1, wherein:

   the polymer gel of the bone implant section and the polymer gel of the artificial cartilage section each include a first mesh structure and a second mesh structure that is entangled with the first mesh structure; and fracture energy of the artificial cartilage implant is 100 J/m$^2$ or more.

5. A method for manufacturing the artificial cartilage implant according to any one of claims 1 to 4, the method comprising:

   preparing a polymer gel; and forming the hydroxyapatite particles in a vicinity of a surface of a region corresponding to the bone implant section by sequentially bringing an alkali metal phosphate aqueous solution and a calcium salt aqueous solution into contact with the region corresponding to the bone implant section while protecting a region corresponding to the artificial cartilage section, the region corresponding to the bone implant and the region corresponding to the artificial cartilage section being in the polymer gel.

6. A method for manufacturing the artificial cartilage implant according to any one of claims 1 to 4, the method comprising:

   preparing a polymer gel; forming the hydroxyapatite particles in a vicinity of a surface of the polymer gel by sequentially bringing an alkali metal phosphate aqueous solution and a calcium salt aqueous solution into contact with the polymer gel; and removing the hydroxyapatite particles in a region corresponding to the artificial cartilage section by bringing an acidic solution into contact with the region corresponding to the artificial cartilage section while protecting a region corresponding to the bone implant section, the region corresponding to the artificial cartilage section and the region corresponding to the bone implant being in the polymer gel after the forming of the hydroxyapatite particles.

10

12

11

FIG. 1A

10

12

11

FIG. 1B

10

12

11

FIG. 1C

10

12

11

FIG. 1D

10

12

100

11

101

FIG. 1E

FIG. 2A

THICKNESS ($\mu$m) OF REGION INCLUDING HYDROXYAPATITE
PARTICLES IN BONE IMPLANT SECTION

FIG. 2B

FIG. 3

## INTERNATIONAL SEARCH REPORT

| International application No. |
|---|
| **PCT/JP2023/020821** |

### A. CLASSIFICATION OF SUBJECT MATTER

*A61L 27/14*(2006.01)i; *A61L 27/12*(2006.01)i; *A61L 27/46*(2006.01)i; *A61L 27/52*(2006.01)i
FI: A61L27/14; A61L27/12; A61L27/46; A61L27/52

According to International Patent Classification (IPC) or to both national classification and IPC

### B. FIELDS SEARCHED

Minimum documentation searched (classification system followed by classification symbols)

A61L27/14; A61L27/12; A61L27/46; A61L27/52

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | PEREIRA, D.R., et al., Injectable gellan-gum/hydroxyapatite-based bilayered hydrogel composites for osteochondral tissue regeneration, Applied Materials Today, 2018, vol. 12, pp. 309-321, ISSN: 2352-9407. | 1, 3 |
| | abstract, p. 310, right column, lines 11-29, p. 314, right column, line 27 to p. 318, left column, line 2, fig. 1, 5, 6 | |
| Y | | 1-6 |
| X | FAN, Z. et al., Gradient Mineralized and Porous Double-Network Hydrogel Effectively Induce the Differentiation of BMSCs into Osteochondral Tissue In Vitro for Potential Application in Cartilage Repair, Macromolecular Bioscience, 2021, vol. 21, no. 3, Article ID: 2000323, pp. 1-10, ISSN: 1616-5195. | 1, 3-4 |
| | abstract, p. 4, right column, line 9 to p. 7, right column, line 8, p. 8, left column, line 17 from the bottom to right column, line 3, fig. 1, 5-7 | |
| Y | | 1-6 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **14 August 2023** | **22 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2023/020821** |

**C.    DOCUMENTS CONSIDERED TO BE RELEVANT**

| Category* | Citation of document, with indication, where appropriate, of the relevant passages | Relevant to claim No. |
|---|---|---|
| Y | WADA, S., et al., Hydroxyapatite-coated double network hydrogel directly bondable to the bone: Biological and biomechanical evaluations of the bonding property in an osteochondral defect, Acta Biomaterialia, 2016, vol. 44, pp. 125-134, ISSN:1742-7061. abstract, p. 126, right column, lines 18-37, p. 128, right column, lines 30-34, fig. 1 | 1-6 |
| A | JP 2021-502140 A (UNIV DUKE) 28 January 2021 (2021-01-28) claims 1–19 | 1-6 |

INTERNATIONAL SEARCH REPORT
Information on patent family members

International application No.

**PCT/JP2023/020821**

| Patent document cited in search report | | | Publication date (day/month/year) | Patent family member(s) | | | Publication date (day/month/year) |
|---|---|---|---|---|---|---|---|
| JP | 2021-502140 | A | 28 January 2021 | US | 2021/0369915 | A1 | |
| | | | | claims 1-19 | | | |
| | | | | WO | 2019/094426 | A1 | |
| | | | | EP | 3706663 | A1 | |
| | | | | KR | 10-2020-0086684 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1305959 A
- JP 2004262976 A
- JP 2004321501 A
- WO 1999058447 A
- JP 2022094413 A

**Non-patent literature cited in the description**

- **TAKAYUKI NONOYAMA**. Double-Network Hydrogels Strongly Bondable to Bones by Spontaneous Osteogenesis Penetration. *Advanced Materials*, 2016, vol. 28, 6740-6745